(19) Europäisches Patentamt
European Patent Office
Office européen des brevets

(11) EP 1 372 611 B1

(12) EUROPEAN PATENT SPECIFICATION

(45) Date of publication and mention of the grant of the patent:
**17.05.2006 Bulletin 2006/20**

(21) Application number: **02732526.5**

(22) Date of filing: **26.03.2002**

(51) Int Cl.:
***A61K 9/19*** *(2006.01)* ***A61K 47/28*** *(2006.01)*
***A61K 47/32*** *(2006.01)* ***A61K 31/553*** *(2006.01)*

(86) International application number:
**PCT/EP2002/003387**

(87) International publication number:
**WO 2002/076432 (03.10.2002 Gazette 2002/40)**

(54) **PHARMACEUTICAL COMPOSITION COMPRISING A POORLY WATER-SOLUBLE STAUROSPORINE, A SURFACTANT AND A WATER-SOLUBLE POLYMER**

PHARMAZEUTISCHE ZUSAMMENSETZUNG ENTHALTEND EIN SCHLECHT WASSERLÖSLICHES STAUROSPORIN, EINE OBERFLÄCHENAKTIVE SUBSTANZ UND EIN WASSERLÖSLICHES POLYMER

COMPOSITION PHARMACEUTIQUE COMPRENANT UN STAUROSPORINE PEU SOLUBLE A L'EAU, UN SURFACTANT ET UN POLYMERE HYDROSOLUBLE

(84) Designated Contracting States:
**AT BE CH CY DE DK ES FI FR GB GR IE IT LI LU MC NL PT SE TR**

(30) Priority: **26.03.2001 DE 10114869**
**05.04.2001 DE 10117049**

(43) Date of publication of application:
**02.01.2004 Bulletin 2004/01**

(73) Proprietors:
- **Novartis AG**
  **4056 Basel (CH)**
  Designated Contracting States:
  **BE CH CY DE DK ES FI FR GB GR IE IT LI LU MC NL PT SE TR**
- **Novartis Pharma GmbH**
  **1230 Wien (AT)**
  Designated Contracting States:
  **AT**

(72) Inventors:
- **EBNER, Andreas**
  **79618 Rheinfelden (DE)**
- **GALLI, Bruno**
  **CH-4411 Seltisberg (CH)**

(56) References cited:
**EP-A- 0 497 162**
**EP-A- 0 733 372**
**EP-A- 1 004 309**
**WO-A-00/48571**
**WO-A-99/04790**
**US-A- 3 754 082**
**US-A- 4 382 953**
**US-A- 4 985 242**
**US-A- 5 599 808**

- **DATABASE WPI Section Ch, Week 198807 Derwent Publications Ltd., London, GB; Class B01, AN 1988-045809 XP002214778 & JP 63 002932 A (TEIJIN LTD), 7 January 1988 (1988-01-07)**
- **H. O. AMMAR: "On the Solubilization Properties of Surfactant-Polymer Complexes" PHARMAZIE 31, vol. 11, 1976, pages 784-786, XP001105403**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 372 611 B1

## Description

The present invention relates to a solid dosage form, for example tablets, coated tablets, capsules or suppositories, based on a pharmaceutical carrier, comprising a solid mixture consisting of a water-soluble basic polymer and an anionic surfactant, or a water-soluble acidic polymer and a cationic surfactant, and a poorly water-soluble staurosporine; a solid mixture consisting of a water-soluble basic polymer and an anionic surfactant, or a water-soluble acidic polymer and a cationic surfactant, and a poorly water-soluble staurosporine; a solution of an anionic surfactant and a water-soluble basic polymer, or a water-soluble acidic polymer and a cationic surfactant, and a poorly water-soluble staurosporine in water, an organic solvent or in a mixture of water and an organic solvent; as well as a method of preparing the solid mixtures. The present invention also relates to aqueous solutions for topical, nasal, parenteral or ophthalmic application.

The poor water solubility and slow rate of dissolution of pharmaceutical active ingredients often linked with this are a serious problem for the pharmacist, since it is extremely difficult to formulate active ingredients of this kind as solid, oral, nasal and rectal, or as liquid, nasal, parenteral or ophthalmic dosage forms with sufficient bioavailability to attain the desired therapeutic effect. In principal active ingredient can only be absorbed in dissolved form. If active ingredients are not dissolved when passing through the gastrointestinal tract, only a fragment of the active ingredient can be absorbed and therefore only a very slight effect or no effect at all can be achieved. If it is not possible to convert such poorly water-soluble active ingredients into dosage forms that can be administered in the required dosage and to create a solution of the active ingredient during passage through the gastrointestinal tract, valuable active substances cannot be used for therapeutical applications.

In Adv. Drug Del. Rev. 25, pages 103-128 (1997), A. J. Humberstone et al. describe a micro-emulsion system comprising surfactants, iipids, co-surfactant and active ingredient, with which individual, poorly water-soluble active ingredients may be processed into an individually dispensed application form by filling it into soft gelatin capsules. WO 00/48571 describes concentrates with N-benzoylstaurosporine, which disperse spontaneously in water into colloids, and which contain a hydrophilic component and a surfactant. They may be processed into orally administrable dosage forms by filling into soft gelatin capsules. The disadvantage is that encapsulation of the liquid system is not standard technology. In addition, these systems do not allow processing into hard gelatin capsules or tablets to take place using standard equipment.

In Pharmazie 31, volume 11, pages 784 to 786 (1976), H. O. Ammar describes experiments on the solubilisation of, for example, p-hydroxyalkyl benzoates in systems comprising sodium dodecyl sulfate (SDS) / polyvinyl pyrrolidone (PVP) / water. In two cases, an increase in solubility over the sole use of SDS is found if PVP is added. These results show that by using SDS and PVP together, the solubility of the said substances in an aqueous medium can be increased. There is however no reference to any solidification of the system or to usages of the aqueous solutions.

EP-B-0 296 110 describes staurosporines which are substituted at the N-methylamino group, and which as selective inhibitors of protein kinase C (PKC) represent valuable pharmaceutical active ingredients.

It has now surprisingly been found that poorly water-soluble active ingredients can be solubilised in sufficient quantities in systems consisting of anionic surfactant / water-soluble basic polymer or cationic surfactant / water-soluble acidic polymer and water. These aqueous systems may be converted into solid substances by removing the water. After adding these solid substances to water, optically clear and stable solutions or an opalescent molecular dispersion, which are not inclined to be supersaturated, are obtained again very rapidly. In particular, these solutions and dispersions are also stable in the pH range of the gastrointestinal tract, so that outstanding bioavailability is attained. The solid substances are therefore eminently suitable for preparing solid and liquid dosage forms, which contain pharmaceutically active amounts of poorly water-soluble pharmaceutical active ingredients, and whose improved solubility assures satisfactory bioavailability. In addition, these solid substances may be prepared in a simple manner with the same or similar characteristics by dissolving the components in an organic solvent and then removing the solvent.

In a first aspect, this invention provides a solid composition comprising

(a) an anionic surfactant in combination with a water-soluble and basic polymer, or (b) a cationic surfactant in combination with a water-soluble and acidic polymer, and

(c) at least one poorly water-soluble pharmaceutical active ingredient which is a staurosporine.

In the context of the invention, poorly water-soluble pharmaceutical active ingredient means that the active ingredient is soluble in one litre of water at 20°C at a rate of less than 100 mg, preferably less than 50 mg, more preferably less than 10 mg, most preferably less than 1 mg.

Many types of anionic surfactants are known. For the composition according to the invention, physiologically acceptable surfactants are chosen. Further surfactants that are preferred are those which form complexes of polymer and surfactant through interaction with the water-soluble polymer, thereby improving the solubility properties of the systems. Surfactants of this type are known. They are primarily organic acids and their physiologically acceptable salts,

for example alkali metal salts (Na or K) or alkaline earth metal salts (Mg or Ca), which contain a hydrophobic substituent. Suitable acids are, for example, carboxylic acids, sulfonic acids, sulfinic acids, phosphonic acids, phosphonous acids, sulfuric acid monoesters, monoesters of sulfurous acid, phosphoric acid mono- or diesters and mono- or diesters of phosphorous acid. Preferred acids are sulfonic acids, phosphonic acids, sulfuric acid monoesters and phosphoric acid mono- or diesters. Sulfuric acid monoesters, and phosphoric acid mono- or diesters are especially preferred.

The acids preferably contain hydrocarbon radicals with at least 6, preferably at least 8 carbon atoms, and up to 30, preferably up to 20 carbon atoms. The hydrocarbon radicals may be interrupted by O, S, CO, -C(O)-O- and/or -C(O)-NH-, and/or unsubstituted or substituted by -OH, -O-$C_1$-$C_{20}$-alkyl, -NH-C(O)-$C_1$-$C_{20}$-alkyl and/or -O-C(O)-$C_1$-$C_{20}$-alkyl The hydrocarbon radicals may be selected from the group linear and branched alkyl; $C_5$-$C_{12}$-cycloalkyl and preferably $C_5$-$C_8$-cycloalkyl substituted by $C_1$-$C_{20}$-alkyl; $C_6$-$C_{10}$-aryl substituted by $C_1$-$C_{20}$-alkyl; and $C_1$-$C_{20}$-alkyl substituted by $C_5$-$C_{12}$-cycloalkyl or $C_8$-$C_{30}$-polycycloalkyl. The polycycloalkyl may be preferably condensed ring systems, as may be found in naturally occurring steroids or bile acids.

Especially preferred anionic surfactants correspond to formulae I and Ia,

R-X (I),

R-C(O)-NH-$R_1$-$SO_3H$ (Ia),

wherein R is a hydrocarbon radical with 6 to 30 carbon atoms, which is optionally interrupted by -O-, -S-, -CO-, -C(O)-O- and/or -C(O)-NH-, and/or is unsubstituted or substituted by -OH, -O-$C_1$-$C_{20}$-alkyl, -NH-C(O)-$C_1$-$C_{20}$-alkyl and/or -O-C(O)-$C_1$-$C_{20}$-alkyl, $R_1$ signifies $C_2$-$C_4$-alkylene, and X is -$SO_3H$ or -$OSO_3H$, as well as the sodium, potassium, magnesium and calcium salts thereof. Examples of surfactants of formula I are $C_6$-$C_{20}$-monoalkylsulfates such as octyl sulfate, decyl sulfate, dodecyl sulfate, tetradecyl sulfate, hexadecyl sulfate and octadecyl sulfate. Examples of surfactants of formula Ia are 1-acylamino-ethane-2-sulfonic acids such as 1-octanoylamino-ethane-2-sulfonic acid, 1-decanoylamino-ethane-2-sulfonic acid, 1-dodecanoylamino-ethane-2-sulfonic acid, 1-teradecanoylamino-ethane-2-sulfonic acid, 1-hexadecanoylamino-ethane-2-sulfonic acid, 1-octadecanoylamino-ethane-2-sulfonic acid, taurocholic acid and taurodesoxycholic acid. Examples of bile acid are cholic acid and desoxycholic acid.

Cationic surfactants are likewise widely known and are available commercially. They are essentially physiologically acceptable onium salts with longer-chained hydrocarbon radicals. Ammonium salts are preferred. The anions of the ammonium salts may be derived from inorganic or organic acids. Examples of anions are chloride, bromide, iodide, sulfate, hydrogensulfate, carbonate, hydrogen carbonate, phospate, formate, acetate and methylsulfonate. The ammonium salts are preferably ammonium salts of primary, secondary and tertiary amines, or quaternary ammonium salts which contain hydrocarbon radicals with at least 8, preferably at least 10 carbon atoms, and up to 30, preferably up to 20 carbon atoms. The hydrocarbon radicals may be interrupted by O, S, CO, -C(O)-O- and/or -C(O)-NH-, and/or unsubstituted or substituted by -OH, -O-$C_1$-$C_{20}$-alkyl, -NH-C(O)-$C_1$-$C_{20}$-alkyl and/or -O-C(O)-$C_1$-$C_{20}$-alkyl The hydrocarbon radicals may be selected from the group linear and branched alkyl; $C_5$-$C_{12}$-cycloalkyl and preferably $C_5$-$C_8$-cycloalkyl substituted by $C_1$-$C_{20}$-alkyl; $C_6$-$C_{10}$-aryl substituted by $C_1$-$C_{20}$-alkyl; and $C_1$-$C_{20}$-alkyl substituted by $C_5$-$C_{12}$-cycloalkyl or $C_8$-$C_{30}$-polycycloalkyl. The polycycloalkyl may be preferably condensed ring systems.

Suitable cationic surfactants for use in the present invention include benzalkonium chloride, cetyldimethylbenzylammonium chloride, cetylammonium chloride, cetrimonium chloride, cetylpyridinium chloride, stearyldimethylbenzyl ammonium chloride, distearyldimethyl ammonium chloride, dodecylpyridinium chloride, laurylpyridinium chloride and myristylpyridinium chloride.

In many cases the dissolving power may be optimised by using anionic and cationic surfactants together. It is similarly possible to add neutral surfactants (alkylated oligomeric polyalkylenediols, alkylated polyols).

Water-soluble polymers are widely known and are available commercially. These may be natural, unmodified or modified polymers, or synthetic polymers. The polymers are selected in such a way that they interact with surfactants, forming molecule complexes. Examples of natural polymers are cellulose, which may be partly alkylated, hydroxyalkylated or acylated, optionally acylated or hydroxyalkylated starch, and peptides. Examples of synthetic polymers are polycarboxamides, for example polylysine or poly(2-ethyl-2-oxazoline), homo- and copolymers of $C_2$-$C_4$-alkylenediols, for example polyethylene glycol, and homo- or copolymers of ethylenically unsaturated monomers with a sufficient proportion of hydrophilic ethylenically unsaturated monomers. The hydrophilic monomers may be, for example, vinyl alcohol, acrylic acid, methacrylic acid, maleic acid, optionally N-alkylated acrylamide or methacrylamide, optionally N-alkylated or acylated vinylamines, for example vinyl pyrrolidone. Possible hydrophobic, ethylenically unsaturated monomers for water-soluble copolymers are, for example, olefins, styrene, acrylates or methacrylates, and vinyl ether. Within the context of the invention, polymers with acidic groups, for example carboxyl groups, are notable as acidic polymers, and polymers with basic groups, for example amine or amide groups, are notable as basic polymers. Water-soluble polymers with OH groups, for example polyalkylene diols, polyvinyl alcohol, cellulose, starch or polymers with predominantly hydrophobic and slightly acidic groups [for example copolymers of (meth)acrylic acid and (meth)acrylic acid alkyl esters or (meth)acrylic acid hydroxyalkyl esters] can interact more strongly with cationic surfactants, and within the context of the invention are classed with the acidic polymers. Polymers with predominantly hydrophobic and slightly basic groups [for example copolymers of (meth)acrylic acid amides and (meth)acrylic acid alkyl esters or (meth)acrylic acid hydroxyalkyl esters] can interact more strongly with anionic surfactants and within the context of the invention are classed with the basic polymers, whereby it may also be appropriate to use cationic surfactants concurrently. The water-soluble polymers may also contain acidic and basic groups, for example copolymers of (meth)acrylic acid and optionally N-alkylated (meth) acrylic acid amides, so that anionic and/or cationic surfactants can be used.

One preferred group of water-soluble and basic polymers is one comprising amide or amine groups in recurring units, since these polymers interact particularly strongly with anionic surfactants. Examples of such polymers are polylysine, polyvinyl pyrrolidone, e.g. PVP K90, PVP K30, PVP K25, PVP K17 or PVP K12, optionally partly or wholly methylated or $C_1$-$C_6$-acylated polyvinyl amines, polyacrylamide, polymethacrylamide, poly-N-methyl- or poly-N-dimethylacryl- or -methacrylamide, and poly(2-ethyl-2-oxazoline). Polyvinyl pyrrolidone is preferred in particular.

The average molecular weight of the water-soluble polymers may be for example 2000 to 2,000,000 and preferably 5000 to 1,000,000 Daltons.

A combination of anionic surfactant and water-soluble polymer which is especially preferred according to the invention is characterised by the choice of $C_6$-$C_{18}$-monoalkylsulfates and their Na+ or K+ salts, and polyvinyl pyrrolidone. Preferred monoalkylsulfates are sodium dodecylsulfate (SDS), sodium decylsulfate and sodium octylsulfate.

Another preferred combination of anionic surfactant and water-soluble polymer is characterised by the choice of bile acids and their Na or K salts, and polyvinyl pyrrolidone. Preferred bile acids are cholic acid, taurocholic acid, taurodesoxycholic acid and glycocholic acid.

Poorly water-soluble pharmaceutical active ingredients are known *per se.* The active ingredients are preferably solid at room temperature. Staurosporine and its derivatives: The biological efficacy of staurosporines is described by D. Fabbro et al. Anti-Cancer Drug Design (2000), 15, pages 17 to 28 (Protein kinase inhibitors with anti-proliferative and antitumour efficacy). One preferred staurosporine corresponds to formula

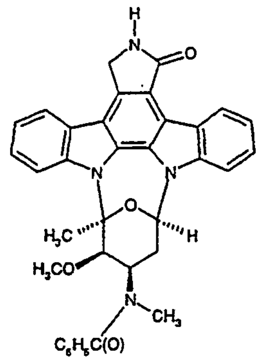

(water solubility: < 0.1 mg/l), which is referred to hereinafter as PKC412.

The weight ratio of water-soluble polymer to anionic or cationic surfactant may be for example 10:1 to 1:1, preferably 5:1 to 1:1 and most preferably 3:1 to 1:1. By having a combination of both components a) and b), the amount used overall as surfactant can be kept within the physiologically acceptable range.

The amount of the poorly water-soluble active ingredient in the solid composition according to the invention may be 0.01 to 30% by weight, preferably 0.01 to 20% by weight, most preferably 0.1 to 15% by weight, based on the anionic and/or cationic surfactant and the water-soluble polymer.

The solid composition according to the invention may exist in the form of powders, finely-dispersed granulates or films.

In another aspect, this invention provides a process for the preparation of the solid composition according to the invention, comprising the steps

a) mixing and dissolving the components (a) anionic surfactant and water-soluble basic polymer, or (b) cationic surfactant and water-soluble acidic polymer and (c) at least one poorly water-soluble staurosporine in water,
b) removing the water until obtaining the solid composition, or
c) mixing and dissolving the components (a) anionic surfactant and water-soluble basic polymer, or (b) cationic surfactant and water-soluble acidic polymer and (c) at least one poorly water-soluble staurosporine in an organic solvent and removing the solvent until obtaining the solid composition.

Typically, the pH may be between 4.5 and 8.0, e.g. between 6 and 7, for example 6.8.

For mixing and dissolving, the components may be added individually, in pairs or all together. Prior to mixing and dissolving in water, it may be expedient to set a specific pH value of the aqueous solution by adding acids, lyes or buffers. Suitable buffers are, for example, phosphate buffers and phosphate/citrate buffers. In order to accelerate the formation of saturated solutions, an excess of components may also be used, after which undissolved portions are filtered off. Dissolving may also be accelerated by heating. The solution is advantageously formed at ca. room temperature to ca. 40°C.

Suitable inert solvents for process stage c) are, for example, aliphatic, cycloaliphatic and aromatic hydrocarbons (pentane, hexane, petroleum ether, cyclohexane, methylcyclohexane, benzene, toluene, xylene), aliphatic halogenated hydrocarbons (methylene chloride, chloroform, di- and tetra-chloroethane), nitriles (acetonitrile, propionitrile, benzonitrile), ethers (diethyl ether, dibutyl ether, t-butylmethyl ether, ethylene glycol dimethyl ether, ethylene glycol diethyl ether, diethylene glycol dimethyl ether, tetrahydrofuran, dioxane, diethylene glycol monomethyl or monoethyl ether), ketones (acetone, methyl isobutyl ketone), carboxylic acid esters and lactones (ethyl or methyl acetate, valerolactone), N-substituted lactams (N-methylpyrrolidone), carboxamides (dimethylamide, dimethylformamide), acyclic ureas (dimethyl imidazoline) and sulfoxides and sulfones (dimethyl sulfoxide, dimethyl sulfone, tetramethylene sulfoxide, tetramethylene sulfone) and alcohols (methanol, ethanol, propanol, butanol, ethylene glycol monomethyl ether, ethylene glycol monoethyl ether, diethylene glycol monomethyl ether) and water. The solvents may be used alone or in a mixture of at least two solvents. Mixtures of solvents also include a mixture of water with at least one organic solvent, for example with ethers or alcohols.

The removal of water or the organic solvent may be carried out in known manner. Suitable methods are evaporation optionally with heating, evaporation under vacuum with optional heating, freeze-drying (lyophilisation), spray drying or spraying onto a carrier in fluid bed.

With the removal of organic solvents according to process stage c) in a container, the solid composition is obtained in the form of films or powders, which, within a short time, dissolve again completely in water or in aqueous buffer solutions, and form slightly opalescent systems which are stable for several days. Examination of the solutions shows that the active ingredient can be partially or completely dissolved, or partially dissolved and the remainder or even the whole amount of the active ingredient can be present in the form of particles with diameters in the submicro range (nanometers to micrometers). In this case, these are highly dispersed systems (molecular or colloidal dispersions), the particles having a diameter in the range of 20 nm to 5 $\mu$m, preferably 30 nm to 2 $\mu$m, most preferably 40 nm to 1 $\mu$m. After administration, absorption of the active ingredient is assured even in the case of the highly dispersed systems, and is fully satisfactory for therapeutical efficacy.

In a further aspect, this invention provides a solution comprising

(a) the anionic surfactant as described above in combination with the water-soluble and basic polymer described herein, or (b) the cationic surfactant as described above in combination with the water-soluble and acidic polymer described herein, and

(c) at least one poorly water-soluble staurosporine in water or an organic solvent.

The combination of the components surfactant and polymer enables considerably larger amounts of the active ingredient to dissolve in the presence of water and optionally buffer, e.g. a phosphate buffer, than each of components a) and b) on its own. The solutions are optically clear and are stable and storable for a longer period of time. The amount of surfactant, polymer and active ingredient in water may be 1 to 30, preferably 5 to 20% by weight, based on the aqueous solution. The amount of surfactant, polymer and active ingredient in an organic solvent may be considerably higher, depending on the dissolving capability of the chosen solvent; the amount may be 1 to 80, preferably 5 to 50% by weight.

The solid compositions according to the invention may be dissolved again in water, optionally in the presence of a buffer, and/or dispersed into a molecular dispersion. These solutions or highly dispersed systems are likewise stable, even in the physiological pH ranges of the gastrointestinal tract. The compositions are therefore eminently suitable for preparing solid dosage forms, which contain therapeutically effective amounts of poorly water-soluble pharmaceutical active ingredients, which are dissolved in the gastrointestinal tract and thus ensure the required bioavailability.

A further object of the invention is solid dosage forms, for example tablets, coated tablets, capsules or suppositories, based on the pharmaceutical carrier, which contain a solid mixture comprising (a) the anionic surfactant as described herein in combination with the water-soluble and basic polymer as described herein, or (b) the cationic surfactant as described herein in combination with the water-soluble and acidic polymer as described herein, and a therapeutically effective amount of poorly water-soluble staurosporine.

The dose is dependent on the physiological efficacy of the active ingredient, as well as on the time interval of the envisaged administration. In general, the amount of active ingredient may be 0.1 to 500 mg, preferably 1 to 100 mg. The solid dosage forms according to the invention also include finely dispersed powders, which can be taken orally or nasally by atomisers, which can be filled as such into capsules, or which can be taken orally after dissolving in water or

in drinks.

Pharmaceutical preparations for oral or rectal administration may be obtained in known manner, by combining the solid composition according to the invention if required with solid carriers, optionally granulating the mixture, optionally adding suitable excipients, and processing this mixture into tablets, tablet cores, capsules, suppositories or powders.

Suitable carriers are in particular fillers, for example sugar (lactose, saccharose, mannitol, sorbitol), cellulose and cellulose derivatives, and/or calcium phosphates (tricalcium phosphate or calcium hydrogen phosphate), binding agents such as starch pastes (for example from corn, wheat, rice or potato starch), gelatin, tragacanth, methyl cellulose, hydroxypropylmethyl cellulose, sodium carboxymethyl cellulose and/or polyvinyl pyrrolidone; and disintegrants, for example starches, carboxymethyl starch, cross-linked polyvinylpyrrolidone, agar, alginic acid or salts thereof. Excipients are primarily flow conditioners and lubricants, for example silicic acid, talc, stearic acid and the magnesium or calcium salts thereof, and polyethylene glycol. Tablet cores are provided with appropriate optionally enteric coatings, using *inter alia* concentrated sugar solutions, optionally comprising gum arabic, talc, polyvinyl pyrrolidone, polyethylene glycol and/or titanium oxide, or coating solutions in suitable organic solvents, or in order to produce enteric coatings, solutions of cellulose preparations, such as ethyl cellulose phthalate or hydroxypropyl methyl cellulose phthalate. The capsules may be hard capsules of hard gelatin or sealed capsules of soft gelatin and a plasticiser, for example glycerol or sorbitol. The hard capsules may contain the composition according to the invention in the form of a powder or granulate, whereby fillers such as lactose, binding agents such as starch, lubricants such as talc or magnesium stearate, and/or stabilisers may optionally be used concurrently. In soft capsules, the active ingredient is advantageously dissolved or suspended in appropriate oily excipients such as paraffin oil or liquid polyethylene glycols, whereby stabilisers and/or antibacterial agents may similarly be added. Dyes and/or pigments can be added to the tablets, tablet coatings and capsule shells, to improve identification. In the preparation of suppositories, fats or oils or other lubricants are frequently added to the carrier in order to increase the gliding ability, before the mass is pressed into its final shape.

The aqueous molecular dispersed to colloidal dispersed solutions according to the invention are suitable, if required using atomisers, for nasal or ophthalmic usage, for topical applications, or for parenteral administration, for example intramuscular or intravenous administration. Isotonic solutions are preferred. Solutions are generally filled into ampoules or vials. Carrier materials may be added to the solutions, for example mannitol. The solutions may be sterilised and may contain excipients, for example electrolyte salts for regulating osmotic pressure, preservatives, stabilisers and wetting agents, physiologically acceptable organic solvents, viscosity-increasing substances (such as sodium carboxymethyl cellulose, carboxymethyl cellulose, dextran, polyvinyl pyrrolidone and gelatin), and buffers. The powders according to the invention, especially lyophilisates, may also be packed *per se* in containers, so that they can be produced just before usage as aqueous and optionally colloidal solutions for nasal or ophthalmic applications, for topical applications, or for parenteral administration, for example intramuscular or intravenous administration.

Following is a description by way of example only of compositions of the invention.

A) Preparation of aqueous solutions and solutions in organic solvents

Example A1:

10 mg/ml of polyvinyl pyrrolidone (PVP K30, BASF), 10 mg/ml of sodium dodecyl sulfate and an excess of PKC412 are added at 25°C to water or phosphate buffer (pH 6.8; 13.96 g $Na_2HPO_4$ x $2H_2O$ and 10.23 g $NaH_2PO_4$ x $2H_2O$ dissolved in 1 l of water) The mixture is stirred for 24 hours, whereby the polymer and the surfactant are completely dissolved, after which the mixture is filtered (Syringe Filter, 0,2 μm). A clear solution is obtained which contains 4.1 mg/ml of PKC412 The solution also remains unchanged after storage for 1 year.

Examples A2-A10:

The procedure of example A1 is followed. The polymers, surfactants and amounts thereof in mg/ml are listed in Table 1. Clear solutions are obtained which are stable and storable.

Table 1:

| Example | polymer (amount in mg/ml) | surfactant (amount in mg/ml) | PKC412 (dissolved amount in mg/ml) |
|---|---|---|---|
| A2 | PVP K30<br>(20) | SDS-Na<br>(10) | (6.1) |
| A3 | PVP K30<br>(40) | SDS-Na<br>(10) | (7.1) |

Table continued

| Example | polymer (amount in mg/ml) | surfactant (amount in mg/ml) | PKC412 (dissolved amount in mg/ml) |
|---|---|---|---|
| A4 | PVP K12 (40) | SDS-Na (20) | (8.4) |
| A5 | PVP K17 (40) | SDS-Na (20) | (9.6) |
| A6 | PVP K25 (40) | SDS-Na (20) | (10.8) |
| A7 | PVP K30 (40) | SDS-Na (20) | (10.8) |
| A8 | PVP K90 (40) | SDS-Na (20) | (10.8) |
| A9 | PVP K30 (40) | Na decyl sulfate (20) | (8.8) |
| A10 | PVP K30 (40) | Na octyl sulfate (20) | (4.9) |
| Abbreviations: PVP is polyvinyl pyrrolidone from BASF. The description K30, K12, etc., refers to the average molecular weight: K30 means 30,000 Daltons, K12 means 12,000 Daltons. | | | |

Example A11:

40 mg/ml of polyvinyl pyrrolidone (PVP K30, BASF), 20 mg/ml of sodium dodecyl sulfate and 8 mg/ml of PKC412 are added to water at 25°C. The mixture is stirred for 24 hours, whereby a clear solution is obtained, which is filtered (Syringe Filter, 0.2 μm). The solution also remains unchanged after storing for 1 year.

Examples A12-A13:

The procedure of example A11 is followed. The polymers, surfactants and amounts thereof in mg/ml are listed in Table 2. Clear solutions are obtained, which are stable and storable.

Table 2:

| Example | polymer (amount in mg/ml) | surfactant (amount in mg/ml) | PKC412 (dissolved amount in mg/ml) |
|---|---|---|---|
| A12 | PVP K30 (80) | SDS-Na (20) | (8.0) |
| A13 | PVP K30 (160) | SDS-Na (20) | (8.0) |

Example A14: Solutions in ethanol

80 mg/ml of polyvinyl pyrrolidone (PVP K30, BASF), 40 mg/ml of sodium taurocholate and 16 mg/ml of PKC412 are dissolved in ethanol at 40°C whilst stirring. A clear and stable solution is obtained.

Examples A15-A17:

The procedure of example A14 is followed. The polymers, surfactants and amounts thereof in mg/ml are listed in Table 3. Clear solutions are obtained, which are stable and storable.

Table 3:

| Example | polymer (amount in mg/ml) | surfactant (amount in mg/ml) | PKC412 (dissolved amount in mg/ml) |
|---|---|---|---|
| A15 | PVP K30 (80) | Na taurocholate (40) | (20.0) |
| A16 | PVP K30 (80) | Na taurocholate (40) | (4.0) |
| A17 | PVP K30 (40) | Na taurocholate (80) | (16.0) |

B) Preparation of solid compositions

Example B1: Freeze-drying in order to produce powders

The solutions according to examples A11-A13 are added to a container to a filling level of ca. 8 mm, and lyophilised in accordance with the adjacent lyophilisation programme in freeze-drying apparatus DELTA 1-24KD (Gefriertrocknungsanlagen Christ, Osterode am Harz, Germany).

A powder is obtained which dissolves again completely in an aqueous medium by shaking gently for less than one minute. For further processing, the powder is passed through a sieve with an average mesh size of 250 μm.

Lyophilisation programme

| Operation | temperature [°C] | pressure [mbar] | time [h] |
|---|---|---|---|
| freezing | -35 | no vacuum | 1 |
| freezing | -35 | no vacuum | 3 |
| main drying | -35 | 1.5 | 1 |
| main drying | -10 | 1.5 | 1 |
| main drying | 0 | 1.5 | 20 |
| after-drying | 0 | 0.4 | 1 |
| after-drying | 0 | 0.4 | 2 |
| after-drying | 25 | 0.4 | 1 |
| after-drying | 25 | 0.4 | 9 |

Example B2:

Evaporation in order to produce powders

Removal of most of the solvent from the solutions according to examples A14-A17 is effected first of all with a Büchi RE 111 rotary evaporator (Büchi Laborgeräte-Technik, Flawil, Switzerland) equipped with a water jet pump. The co-evaporates are then pre-dried for ca. 12 hours at 40°C and 25 - 10 mbar in a vacuum drier (Salvis, Rotkreuz, Switzerland) and afterwards passed through a sieve with an average mesh size of 500 μm. Final drying is effected again under the above-mentioned conditions until reaching a constant weight.

C) Preparation of capsules and tablets

Example C1: Preparation of capsules

A powder is used, which was produced according to example A11 and subsequently dried in accordance with example B1. A corresponding quantity, which contains 25 mg of PKC412, is filled in a transparent, colourless hard gelatin capsule of capsule size 00 (Capsugel, from Bornem in Belgium).

Example C2: Preparation of capsules

A powder is used, which was produced according to example A14 and subsequently dried in accordance with example B2. A corresponding quantity, which contains 25 mg of PKC412, is filled in a transparent, colourless hard gelatin capsule of capsule size 1 (Capsugel, from Bornem in Belgium).

Example C3: Precompaction of powders

Precompaction is effected with a tabletting machine EK 0 (Korsch, Berlin). A check of the breaking strength is carried out using a Tablet Tester 6D (Schleuniger, Solothurn, Switzerland).
The powder which is produced according to Example A11 with subsequent treatment according to Example B1 is compressed into an intermediate press-cake with a breaking strength of ca. 10 -15 N and then broken over a sieve with an average mesh size of 1 mm.

Example C4-C5: Preparation of tablets

Preparation of tablets is effected with a tabletting machine EK 0 (Korsch, Berlin). A check of the breaking strength is carried out using a Tablet Tester 6D (Schleuniger, Solothurn, Switzerland).
The powder of Example C3 is used as the active ingredient-containing powder.
The corresponding amounts of active ingredient-containing powder, microcrystalline cellulose and crosslinked polyvinyl pyrrolidone according to Table 4 are premixed by a Tubula mixer for 5 minutes at 50 rpm. Afterwards, the mixture is sifted through a sieve of mesh size 1 mm. Then, the amount of magnesium stearate of Table 5 is added and mixing continues for a further 5 minutes. This mixture is pressed into round tablets with a diameter of 9 mm, a breaking strength of 35-40 N and a weight of 320 mg.

Table 5: Composition of tablets with a theoretical content of 25 mg PKC412

| Example | active ingredient-containing powder (mg per tablet) | microcrystalline cellulose (mg per tablet) | crosslinked polyvinyl pyrrolidone (mg per tablet) | magnesium stearate (mg per tablet) |
|---|---|---|---|---|
| C4 | 212.5 | 104.3 | 0.0 | 3.2 |
| C5 | 196.5 | 88.3 | 16.0 | 3.2 |

Example C6: Preparation of tablets

Preparation of tablets having a single dose of 25 mg PKC412 is effected with a tabletting machine EK 0 (Korsch, Berlin). A check of the breaking strength is carried out using a Tablet Tester 6D (Schleuniger, Solothurn, Switzerland). The active ingredient-containing powder used is a powder produced according to example A14 with subsequent treatment according to example B2.
The corresponding amounts of active ingredient-containing powder and microcrystalline cellulose according to Table 6 are premixed by a Tubula mixer for 5 minutes at 50 rpm. Afterwards, the mixture is sifted through a sieve of mesh size 0.5 mm. Then, the amount of magnesium stearate of Table 6 is added and mixing continues for a further 5 minutes. This mixture is pressed into round tablets with a diameter of 12 mm, a breaking strength of 35-40 N and a weight of 500 mg.

Table 6: Composition of tablets with a theoretical content of 25 mg PKC412

| Example | active ingredient-containing powder (mg per tablet) | microcrystalline cellulose (mg per tablet) | magnesium stearate (mg per tablet) |
|---|---|---|---|
| C6 | 212.5 | 282.5 | 5.0 |

D) Application examples

Example D1: Dissolution behaviour of capsules

The medium used for the dissolution behaviour test is 1 litre of a surfactant-free, modified medium according to "Intestinal Fluid, Simulated, TS" from USP XXIV ($SIF_{mod}$). $KH_2PO_4$ is replaced by $NaH_2PO_4$. Pancreatin is not added (pH 6.8).
The dissolution test is carried out according to the "Paddle method" of USP XXIV at 37°C and at a stirring rate of 50 rpm with a Sotax AT6 (Sotax, Basle, Switzerland). The capsules are placed in a teflon-coated sinker (ATN, Pfaffenheim, France), in order to allow them to remain on the bottom of the dissolution container. The comparable solubility of untreated PKC412 based on a 25 mg dosage is < 0.4% of the theoretical content.
The average amounts of active ingredient released from each of 3 capsules of 25 mg PKC412 according to examples C1 and C2 are given in Table 7 as a percentage of the theoretical content.

Table 7: Release from 25 mg PKC412 capsules in $SIF_{mod}$ after different intervals as a percentage of the theoretical content (n = 3)

| capsules according to example | 10 mins | 15 mins | 20 mins | 30 mins | 45 mins | 60 mins |
|---|---|---|---|---|---|---|
| C1 | 23 | 53 | 73 | 92 | 95 | 95 |
| C2 | 33 | 65 | 86 | 93 | 93 | 93 |

Example D2: Dissolution behaviour of tablets

The medium used for the dissolution behaviour test is 1 litre of a surfactant-free, modified medium according to "Intestinal Fluid, Simulated, TS" from USP XXIV ($SIF_{mod}$). $KH_2PO_4$ is replaced by $NaH_2PO_4$. Pancreatin is not added (pH 6.8).
The dissolution test is carried out according to the "Paddle method" of USP XXIV at 37°C and at a stirring rate of 50 rpm with a Sotax AT6 (Sotax, Basle, Switzerland). The tablets were added and the amounts of active ingredient released were analysed. The comparable solubility of untreated PKC412 based on a 25 mg dosage is < 0.4% of the theoretical content.
The average amounts of active ingredient released from each of 3 tablets of 25 mg PKC412 according to examples C4-C6 are given in Table 8 as a percentage of the theoretical content.

Table 8: Release from 25 mg PKC412 tablets in $SIF_{mod}$ after different intervals as a percentage of the theoretical content (n = 3)

| tablets according to example | 10 mins | 15 mins | 20 mins | 30 mins | 45 mins | 60 mins |
|---|---|---|---|---|---|---|
| C4 | 32 | 48 | 61 | 80 | 95 | 96 |
| C5 | 28 | 39 | 51 | 72 | 93 | 98 |
| C6 | 37 | 53 | 67 | 79 | 92 | 95 |

Example E1: Bioavailability tests

In a relative bioavailability study, the capsules according to example C1 and C2 are tested against a liquid, spontaneously dispersing formulation in soft gelatin capsules (reference). The study is carried out in the Crossover Design with 9 male Beagle dogs (age: 1-11 years, weight: 9-12 kg).
Each dog is given two capsules of each formulation as a single dose (corresponding to 50 mg PKC412) in the pharynx and rinsed down with ca. 20 ml of demineralised water. Blood samples are taken before and 15 mins, 30 mins, 45 mins, 1 h, 1.5 h, 2 h, 4 h, 6 h, 10 h, 24 h, 30 h and 48 h after administration and the concentration of PKC412 in the blood plasma is determined. The results of this bioavailability study are listed in table 9.

Table 9: Results of the bioavailability study of the capsules according to example C1 and C2 against a spontaneously dispersing formulation in soft gelatin capsules (reference). These are average values and the CV% is given in parenthesis (n = 9).

| | (reference) | capsules according to example C1 | capsules according to example C2 |
|---|---|---|---|
| Dose (mg/kg) | 4.51 (8) | 4.52 (9) | 4.50 (10) |
| AUC(0-48h) [(ng/ml) x h] | 3698 (24) | 2435 (49) | 3405 (31) |
| AUC(0-48h)/dose [(ng/ml) x h/(mg/kg)] | 813 (26) | 536 (53) | 739 (28) |
| $c_{max}$ [ng/mL] | 544 (19) | 253 (56) | 473 (29) |
| $c_{max}$/dose [(ng/mL)/(mg/kg)] | 119 (23) | 55.5 (58) | 102 (24) |
| $t_{max}$ [h] | 1.4 (27) | 1.8 (20) | 1.5 (29) |
| $F_{rel}$ [%] | 100 | 66.4 (45) | 93.2 (24) |
| $F_{rel}$ range [%] | | 31 to 127 | 68 to 132 |

The maximum plasma concentration ($c_{max}$) in all formulations is reached very quickly after 1 to 2 hours ($t_{max}$). The other pharmacokinetic parameters ($c_{max}$/dose, AUC (0-48 h)/dose) indicate that the absorption of PKC412 with capsules according to example C2 and the reference are very similar, but with capsules according to example C1 is likewise high, but slightly lower. In addition, the variability of $c_{max}$/dose or AUC (0-48 h)/dose with capsules according to example C1 is higher than with the other two capsules. The sequence of bioavailabilities of the three capsule formulations is:

reference ≅ capsules according to C2 > capsules according to C1

To summarise, the poorly water-soluble active ingredient PKC412 can be processed into solid powders with polymers and surfactants using various processes, and after contact with an aqueous medium, these powders can rapidly disperse the active ingredient PKC412 and maintain it in dissolved form. The powders enable further processing to take place into capsules or tablets, which *in vitro* exhibit rapid release of the active ingredient and *in vivo* in the dog can have comparable bioavailability to a spontaneously dispersing formulation in soft gelatin capsules (reference).

## Claims

1. A solid composition comprising

   (a) an anionic surfactant in combination with a water-soluble and basic polymer, or (b) a cationic surfactant in combination with a water-soluble and acidic polymer, and
   (c) at least one poorly water-soluble staurosporine,

   wherein the staurosporine is soluble in one litre of water at 20°C at a rate of less than 10 mg.

2. A composition according to claim 1, wherein the anionic surfactant is selected from organic acids and their physiologically acceptable salts, which contain a hydrophobic substituent, and the cationic surfactant is selected from physiologically acceptable onium salts with longer-chained hydrocarbon radicals.

**3.** A composition according to claim 1, wherein the water-soluble and basic polymer comprises amide or amine groups in recurring units.

**4.** A composition according to claim 3, wherein the polymer is selected from the group polylysine, polyvinyl pyrrolidone, optionally partly or wholly methylated or $C_1$-$C_6$-acylated polyvinyl amines, polyacrylamide, polymethacrylamide, poly-N-methyl- or poly-N-dimethylacryl- or -methacrylamide, and poly(2-ethyl-2-oxazoline).

**5.** A composition according to claim 1, wherein the water-soluble basic polymer is polyvinyl pyrrolidone.

**6.** A composition according to claim 1, wherein the anionic surfactant is a $C_6$-$C_{18}$-monoalkyl sulfate or Na+ or K+ salt thereof, and the water-soluble basic polymer is polyvinyl pyrrolidone.

**7.** A composition according to claim 1, wherein the poorly water-soluble pharmaceutically active ingredient is a staurosporine of formula

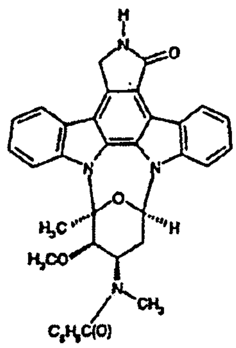

**8.** A composition according to claim 1, wherein the weight ratio of water-soluble polymer to anionic or cationic surfactant is 10:1 to 1:1.

**9.** A composition according to claim 1, wherein the amount of poorly water-soluble active ingredient is 0.01 to 30% by weight based on the anionic or cationic surfactant and the water-soluble polymer.

**10.** A composition according to claim 1, which is present in the form of powders, finely-dispersed granulates or films.

**11.** A process for the preparation of the solid composition according to claim 1, comprising the steps

a) mixing and dissolving the components (a) anionic surfactant and water-soluble basic polymer, or (b) cationic surfactant and water-soluble acidic polymer, and (c) at least one poorly water-soluble staurosporine in water,
b) removing the water until the solid composition is obtained, or
c) mixing and dissolving the components (a) anionic surfactant and water-soluble basic polymer, or (b) cationic surfactant and water-soluble acidic polymer, and (c) at least one poorly water-soluble staurosporine in an organic solvent and removing the solvent until the solid composition is obtained.

**12.** A solution comprising

(a) an anionic surfactant in combination with a water-soluble and basic polymer, or (b) a cationic surfactant in combination with a water-soluble and acidic polymer, and
(c) at least one poorly water-soluble staurosporine in water or in an organic solvent, wherein the staurosporine is soluble in one litre of water at 20°C at a rate of less than 10 mg.

**13.** Aqueous solution according to claim 12 for ophthalmic, nasal or parenteral application.

**14.** Solid dosage forms based on a pharmaceutical carrier, which contain a solid mixture consisting of (a) an anionic surfactant in combination with a water-soluble and basic polymer, or (b) a cationic surfactant in combination with a water-soluble and acidic polymer, and a therapeutically effective amount of a poorly water-soluble staurosporine, wherein the staurosporine is soluble in one litre of water at 20°C at a rate of less than 10 mg.

**Patentansprüche**

1. Feste Zusammensetzung, die umfasst

   (a) ein anionisches Tensid in Kombination mit einem wasserlöslichen und basischen Polymer oder
   (b) ein kationisches Tensid in Kombination mit einem wasserlöslichen und sauren Polymer und
   (c) zumindest ein schlecht wasserlösliches Staurosporin,

   worin das Staurosporin in 1 Liter Wasser bei 20°C mit einer Menge von weniger als 10 mg löslich ist.

2. Zusammensetzung nach Anspruch 1, worin das anionische Tensid aus organischen Säuren und ihren physiologisch annehmbaren Salzen ausgewählt ist, die einen hydrophoben Substituenten enthalten und das kationische Tensid aus physiologisch annehmbaren Oniumsalzen mit längerkettigen Kohlenwasserstoffresten ausgewählt ist.

3. Zusammensetzung nach Anspruch 1, worin das wasserlösliche und basische Polymer Amid- oder Amingruppen in wiederkehrenden Einheiten aufweist.

4. Zusammensetzung nach Anspruch 3, worin das Polymer aus der Gruppe ausgewählt ist, die besteht aus Polylysin, Polyvinylpyrrolidon, wahlweise partiell oder vollkommen methylierten oder $C_1$-$C_6$-acylierten Polyvinylaminen, Polyacrylamid, Polymethacrylamid, Poly-N-methyl- oder Poly-N-dimethylacryl- oder -methacrylamid und Poly(2-ethyl-2-oxazolin).

5. Zusammensetzung nach Anspruch 1, worin das wasserlösliche basische Polymer Polyvinylpyrrolidon ist.

6. Zusammensetzung nach Anspruch 1, worin das anionische Tensid ein $C_6$-$C_{18}$ Monoalkylsulfat oder $Na^+$ oder $K^+$ Salz hiervon ist und das wasserlösliche basische Polymer Polyvinylpyrrolidon ist.

7. Zusammensetzung nach Anspruch 1, worin der schlecht wasserlösliche pharmazeutische Wirkstoff ein Staurosporin der folgenden Formel ist

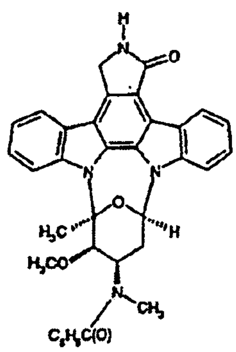

8. Zusammensetzung nach Anspruch 1, worin das Gewichtsverhältnis an wasserlöslichem Polymer zu anionischem oder kationischem Tensid 10:1 bis 1:1 beträgt.

9. Zusammensetzung nach Anspruch 1, worin die Menge an schlecht wasserlöslichem Wirkstoff 0,01 bis 30 Gewichtsprozent auf der Grundlage des anionischen oder kationischen Tensids und des wasserlöslichen Polymers beträgt.

10. Zusammensetzung nach Anspruch 1, die in Form von Pulvern, fein dispergierten Granula oder Filmen vorkommt.

11. Verfahren zur Herstellung der festen Zusammensetzung nach Anspruch 1, das die folgenden Schritte umfasst

    a) Mischen und Lösen der folgenden Komponenten (a) anionisches Tensid und wasserlösliches basisches Polymer oder (b) kationisches Tensid und wasserlösliches saures Polymer und (c) zumindest ein schlecht wasserlösliches Staurosporin in Wasser,
    b) Entfernung des Wassers bis zum Erhalt der festen Zusammensetzung, oder
    c) Mischen und Lösen der folgenden Komponenten (a) anionisches Tensid und wasserlösliches basisches

Polymer oder (b) kationisches Tensid und wasserlösliches saures Polymer und (c) zumindest ein schlecht wasserlösliches Staurosporin in einem organischen Lösemittel und Entfernen des Lösemittels bis zum Erhalt der festen Zusammensetzung.

**12.** Lösung, die umfasst

(a) ein anionisches Tensid in Kombination mit einem wasserlöslichen und basischen Polymer oder (b) ein kationisches Tensid in Kombination mit einem wasserlöslichen und sauren Polymer und (c) zumindest ein schlecht wasserlösliches Staurosporin in Wasser oder in einem organischen Lösemittel, worin das Staurosporin in 1 Liter Wasser bei 20°C in einer Menge von weniger als 10 mg löslich ist.

**13.** Wässrige Lösung nach Anspruch 12 für eine ophthalmologische, nasale oder parenterale Anwendung.

**14.** Feste Dosierungsformen, die auf einem pharmazeutischen Träger basieren, der ein festes Gemisch enthält, das besteht aus (a) einem anionischen Tensid in Kombination mit einem wasserlöslichen und basischen Polymer oder (b) einem kationischen Tensid in Kombination mit einem wasserlöslichen und sauren Polymer und einer therapeutisch wirksamen Menge eines schlecht wasserlöslichen Staurosporins, worin das Staurosporin in 1 1 Wasser bei 20°C in einer Menge von weniger als 10 mg löslich ist.

**Revendications**

**1.** Composition solide comprenant

(a) un tensioactif anionique en combinaison avec un polymère basique hydrosoluble, ou
(b) un tensioactif cationique en combinaison avec un polymère acide hydrosoluble, et
(c) au moins une staurosporine faiblement hydrosoluble,

**caractérisée en ce que** la staurosporine est soluble dans un litre d'eau à 20°C lorsqu'elle est employée selon un taux inférieur à 10 mg.

**2.** Composition selon la revendication 1, **caractérisée en ce que** le tensioactif anionique est sélectionné à partir d'acides organiques et de leurs sels physiologiquement acceptables qui contiennent un substituant hydrophobe et **en ce que** le surfactant cationique est sélectionné à partir de sels d'onium physiologiquement acceptables dotés de radicaux hydrocarbures à plus longues chaînes.

**3.** Composition selon la revendication 1 **caractérisée en ce que** le polymère basiques hydrosoluble comprend des groupes amides ou amines dans des unités récurrentes.

**4.** Composition selon la revendication 3, **caractérisée en ce que** le polymère est sélectionné à partir du groupe composé de polylysine, polyvinylpyrrolidone, amines polyvinyliques partiellement ou entièrement méthylées ou $C_1$-$C_6$-acylées, polyacrylamide, polyméthacrylamide, poly-N-méthyl- ou poly-N-diméthylacryl- ou -méthacrylamide et poly(2-éthyl-2-oxazoline).

**5.** Composition selon la revendication 1, **caractérisée en ce que** le polymère basique hydrosoluble est de la polyvinylpyrrolidone.

**6.** Composition selon la revendication 1, **caractérisée en ce que** le tensioactif anionique est un $C_6$-$C_{18}$-monoalkylsulfate ou un de ses sels $Na^+$ ou $K^+$ et **en ce que** le polymère basique hydrosoluble est de la polyvinylpyrrolidone.

**7.** Composition selon la revendication 1, **caractérisée en ce que** l'ingrédient pharmaceutiquement actif faiblement hydrosoluble est une staurosporine de formule

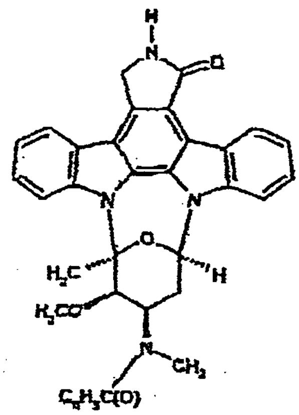

**8.** Composition selon la revendication 1, **caractérisée en ce que** le rapport pondéral entre le polymère hydrosoluble et le tensioactif anionique ou cationique est compris dans la gamme allant de 10 : 1 à 1 : 1.

**9.** Composition selon la revendication 1, **caractérisée en ce que** la quantité de l'ingrédient actif faiblement hydrosoluble est comprise dans la gamme allant de 0,01 à 30% en poids en fonction du tensioactif anionique ou cationique et du polymère hydrosoluble.

**10.** Composition selon la revendication 1 qui se présente sous forme de poudres, de granulés finement dispersés ou de films.

**11.** Procédé de préparation d'une composition solide selon la revendication 1 consistant :

a) à mélanger et dissoudre les composants comprenant (a) un tensioactif anionique et un polymère basique hydrosoluble, ou (b) un tensioactif cationique et un polymère acide hydrosoluble, et (c) au moins une staurosporine faiblement hydrosoluble dans l'eau,
b) à extraire l'eau jusqu'à l'obtention d'une composition solide, ou
c) à mélanger et dissoudre les composants comprenant (a) un tensioactif anionique et un polymère basique hydrosoluble, ou (b) un tensioactif cationique et un polymère acide hydrosoluble, et (c) au moins une staurosporine faiblement hydrosoluble dans un solvant organique et à extraire le solvant jusqu'à l'obtention d'une composition solide.

**12.** Solution comprenant

(a) un tensioactif anionique en combinaison avec un polymère basique hydrosoluble, ou (b) un tensioactif cationique en combinaison avec un polymère acide hydrosoluble, et
(c) au moins une staurosporine faiblement hydrosoluble dans l'eau ou dans un solvant organique **caractérisé en ce que** la staurosporine est soluble dans un litre d'eau à 20°C lorsqu'elle est employée selon un taux inférieur à 10 mg.

**13.** Solution aqueuse selon la revendication 12 destinée à une application ophtalmique, nasale ou parentérale.

**14.** Formes de dosage solides basées sur un véhicule pharmaceutique contenant un mélange solide composé (a) d'un tensioactif anionique en combinaison avec un polymère basique hydrosoluble ou (b) d'un tensioactif cationique en combinaison avec un polymère acide hydrosoluble, et une quantité thérapeutiquement efficace d'une staurosporine faiblement hydrosoluble **caractérisées en ce que** la staurosporine est soluble dans un litre d'eau à 20°C lorsqu'elle est employée selon un taux inférieur à 10 mg.